# EUROPÄISCHE PATENTSCHRIFT

(11) **EP 2 106 545 B1**
(45) Veröffentlichungstag und Bekanntmachung des Hinweises auf die Patenterteilung: **22.07.2015**
(21) Anmeldenummer: 07857438.1
(22) Anmeldetag: 12.12.2007
(51) Int. Cl.: G01N 27/407, G01N 33/00

(54) **FESTELEKTROLYT-SENSORELEMENT ZUR BESTIMMUNG VON AMMONIAK IN EINEM MESSGAS**
SOLID-ELECTROLYTE SENSOR ELEMENT FOR DETERMINING AMMONIA IN A MEASUREMENT GAS
ÉLÉMENT DE DÉTECTION À ÉLECTROLYTE SOLIDE POUR DÉTERMINER LA CONCENTRATION D'AMMONIAC DANS UN GAZ À MESURER

(30) Priorität: 29.12.2006 DE 102006062058
(43) Veröffentlichungstag der Anmeldung: 07.10.2009
(73) Patentinhaber: Robert Bosch GmbH, 70442 Stuttgart (DE)
(72) Erfinder: ZIEGLER, Joerg, 71277 Rutesheim (DE); ROESSLER, Mario, 70176 Stuttgart (DE); SCHUMANN, Bernd, 71277 Rutesheim (DE); CRAMER, Berndt, 71229 Leonberg (DE)
(86) Internationale Anmeldenummer: PCT/EP2007/063772
(87) Internationale Veröffentlichungsnummer: WO 2008/080781

(56) Entgegenhaltungen:
- DE-A1- 10 048 240
- DE-A1- 10 106 171
- DE-A1- 10 121 771
- DE-A1- 10 147 408
- DE-A1- 10 325 648
- DE-A1- 10 346 904
- DE-A1- 19 837 074

## Beschreibung

### Stand der Technik

Die Erfindung geht aus von einem Sensorelement zur Bestimmung der Konzentration von Ammoniak in einem Messgas, insbesondere im Abgas einer Brennkraftmaschine, nach dem Oberbegriff des Anspruchs 1.

Bei Betriebsbedingungen, die einem Luft-Kraftstoffverhältnis von λ=1 entsprechen, werden im Abgas enthaltene Stickoxide im Abgaskatalysator weitestgehend durch ebenfalls im Abgas vorhandene, reduzierende Komponenten, wie beispielsweise Kohlenwasserstoffe, zu Stickstoff, Wasser und Kohlendioxid umgesetzt. Im Magerbetrieb mit einer Luftzahl λ>1 steht dagegen keine ausreichende Menge an reduzierenden Komponenten im Abgas zur Verfügung, so dass überschüssige Stickoxide auf anderem Weg beseitigt werden. Eine bekannte Methode ist die gezielte Zudosierung vom Ammoniak oder ammoniakerzeugenden Substanzen in den Abgasstrom. Dies erfolgt in Richtung des Abgases vor einem weiteren Katalysator, an dessen Oberfläche die Reaktion der Stickoxide mit Ammoniak zu Stickstoff und Wasser abläuft. Um diese sog. SCR-Methode (Selective Catalytic Reduction Method) effektiv anwenden zu können, muss die zudosierte Menge an Ammoniak möglichst exakt dem Überschuss an Stickoxiden angepasst sein. Dafür werden auf Ammoniak empfindliche und selektive Gassensoren verwendet, mittels derer der Ammoniakgehalt im Abgas bestimmt werden kann.

Ein bekanntes Sensorelement für einen Gassensor zur Bestimmung der Konzentration von in einem Gasgemisch vorhandenem Wasserstoff oder einer wasserstoffhaltigen Gaskomponente, vorzugsweise Ammoniak (NH₃), (DE 199 63 008 A1) weist eine einem Gasgemisch ausgesetzte Messelektrode und eine einem Referenzgas ausgesetzte Referenzelektrode auf, die auf voneinander abgekehrten Seiten einer protonenleitenden Festelektrolytschicht angeordnet sind. Mehrere Festelektrolytschichten sind zu einem keramischen Körper zusammenlaminiert, wobei die Referenzelektrode in einem zwischen den Festelektrolytschichten ausgebildeten Referenzgasraum einliegt. Alle protonenleitenden Festelektrolytschichten bestehen beispielsweise aus Ceroxid (CeO₂) mit Dotierungen von Erdalkalioxiden, z.B. Kalziumoxid (CAO), Strontiumoxid (SrO), Bariumoxid (BaO). Die ammoniaksensitive Messelektrode besteht aus einem katalytisch inaktiven Material, wie Gold, Palladium, Silber oder Rhutenium. Die Referenzelektrode ist aus einem katalytisch aktiven Material, z.B. Platin. In dem keramischen Körper ist ein in einer elektrischen Isolierung eingebetteter, elektrischer Widerstandheizer angeordnet. Der Widerstandheizer dient zum Aufwärmen des Sensorelements auf die notwendige Betriebstemperatur von ca. 500°C. Zur Bestimmung des Ammoniakgehalts im Gasgemisch werden Mess- und Referenzelektrode als sog. Nernstzelle betrieben, wobei die durch unterschiedliche Konzentrationen von Wasserstoff- bzw. Protonen an Mess- und Referenzelektrode entstehende, elektromotorische Kraft (EMK) als Spannung gemessen wird. Die Höhe der Spannung ist ein Maß für die Wasserstoff- bzw. Protonenkonzentration an der Messelektrode und damit ein Maß für den Ammoniakgehalt im Messgas. Aufgrund der Verwendung eines protonenleitenden Festelektrolyten hat das Sensorelement praktisch keine Querempfindlichkeit zu sauerstoffhaltigen Verbindungen, wie den Stickoxiden.

Ein ebenfalls bekanntes Sensorelement für einen Gassensor zur Bestimmung der Konzentration von Ammoniak in einem Gasgemisch (EP 1 452 860 A1) weist mindestens eine Hilfselektrode und mindestens eine in Strömungsrichtung des Gasgemisches nachgeordnete Messelektrode auf, die in direktem Kontakt mit dem Gasgemisch stehen. Mittels der Messelektrode wird zumindest zeitweise ein Signal zur Bestimmung der Konzentration von Ammoniak generiert. Hierzu wird an die Hilfselektrode ein Potenzial angelegt, bei dem lediglich Sauerstoff bzw. Stickoxide reduziert und aus dem Gasgemisch entfernt werden. An der Masseelektrode liegt ein Potenzial, bei dem im Gasgemisch vorhandenes Ammoniak oxidiert wird. Der Pumpstrom von der Messelektrode zur Referenzelektrode wird als Maß für die Konzentration an Ammoniak im Gasgemisch herangezogen. Mittels einer zwischen der Hilfselektrode und der Messelektrode angeordneten zweiten Hilfselektrode wird im Gasgemisch noch vorhandenes Ammoniak zu entsprechenden Stickoxiden, insbesondere Stickstoffmonoxid, oxidiert. Gleichzeitig wird die im Gasgemisch noch vorhandene Sauerstoffkonzentration weiter verringert und im Gasgemisch enthaltener Wasserstoff oxidiert. Auf diese Weise zeigt die Messelektrode eine geringe Empfindlichkeit gegenüber dem Wasserstoffgehalt des zu messendes Gasgemisches.

DE 101 06 171 offenbart ein Verfahren, das dem Nachweis der Konzentration von Ammoniak dient und der Gassensor in einem Abgasstrang einem NOx-Speicherkatalysator in Strömungsrichtung des Abgases nachgeordnet angebracht ist.

Die Dokumente DE 101 21 771 A1 und DE 100 48 240 A1 offenbaren NOx -Sensorelemente mit Mitteln zur Absorption von Stickoxiden.

DE 103 46 904 A1 offenbart einen NOx -Sensor, wobei ein NOx -Speicherkatalysator getrennt vom Sensor im Abgasstrang untergebracht ist.

DE 101 47 408 A1 offenbart ein Verfahren zur Regenerierung eines NOx /NH3 -Gassensors, wobei der Gassensor eine Schutzschicht mit Getterstoffen umfasst. Diese Getterstoffe sind jedoch so beschaffen, dass sie NOx nicht zurückhalten.

### Offenbarung der Erfindung

Das erfindungsgemäße Sensorelement mit den Merkmalen des Anspruchs 1 hat den Vorteil, dass nicht die Empfindlichkeit der Messelektroden bzw. Messstrecke gegenüber Sauerstoffverbindungen, insbesondere Stickoxiden, selbst reduziert wird, sondern die störenden Stickoxide aus dem Abgas entfernt werden, bevor das Messgas an die Messelektrode bzw. in die Messstrecke gelangt. Dies ergibt eine vollständige Unempfindlichkeit des Sensorelements gegenüber Stickoxiden, die lediglich abhängig ist von der Güte der Stickstoffoxidabsorption in dem an die Messelektrode gelangenden Messgasvolumen. Die Eliminierung der Stickoxide erfolgt durch Einlagerung in das vorzugsweise barium- oder bariumoxidhaltige Absorptionsmittel als Bariumnitrat (Ba(NO₃)₂). Das Absorptionsmittel lässt sich durch verschiede Methoden regenerieren.

Durch die in den weiteren Ansprüchen aufgeführten Maßnahmen sind vorteilhafte Weiterbildungen und Verbesserungen des im Anspruch 1 angegebenen Sensorelements möglich.

Gemäß einer vorteilhaften Ausführungsform der Erfindung wird als Absorptionsmittel ein regenerierbarer Stickoxid-Speicher aus porösem Material mit einer bariumhaltigen Speicherkomponente und vorzugsweise einer zusätzlichen Edelmetallkomponente zur Oxidation von Stickstoffmonoxid (NO) zu Stickstoffdioxid (NO₂), da letzteres sehr viel besser gespeichert werden kann. Das Barium in der Speicherkomponente liegt in Form von Bariumoxid (BaO) oder Bariumkarbonat (BaCO₃) vor, das zur Speicherung der Stickoxide in Bariumnitrat (Ba(NO₃)₂) umgewandelt wird.

Gemäß einer vorteilhaften Ausführungsform der Erfindung wird zur Regeneration des Stickoxid-Speichers, letzterer einer Temperatur ausgesetzt, die größer 500°C ist. Bei dieser Temperatur wird das Bariumnitrat wieder zu Bariumoxid zersetzt, so dass der Stickoxid-Speicher wieder voll aufnahmefähig ist.

Gemäß einer alternativen Ausführungsform der Erfindung wird zur Regeneration des Stickoxid-Speichers in dem Bereich zwischen Stickoxid-Speicher und Messelektrode ein Fettgas erzeugt, indem hier durch Anlegen eines entsprechenden Spannungspotenzials an die Messelektrode eine Zersetzung des im Messgas enthaltenen Wassers und Kohlendioxid zu Wasserstoff und Kohlenmonoxid herbeigeführt wird. Das Kohlenmonoxid reagiert mit dem gespeicherten Bariumnitrat und wandelt es in Bariumoxid und/oder Bariumkarbonat um. Die dabei freiwerdenden, negativ geladenen Sauerstoffionen werden aufgrund des herrschenden Spannungspotenzials von der Messelektrode abtransportiert.

Gemäß einer vorteilhaften Ausführungsform der Erfindung ist die Messelektrode in einem Messraum angeordnet, der einen Messgaszutritt aufweist, und der Stickoxid-Speicher im Messgaszutritt angeordnet. Ein die Betriebstemperatur der Messelektroden einstellender elektrischer Heizer ist relativ zu Messraum und Messgaszutritt so angeordnet, dass bei Betriebstemperatur an der Messelektrode der in dem Messgaszutritt angeordnete Stickoxid-Speicher eine optimale Speichertemperatur aufweist, die vorzugsweise zwischen 200°C bis 400°C liegt.

### Kurze Beschreibung der Zeichnungen

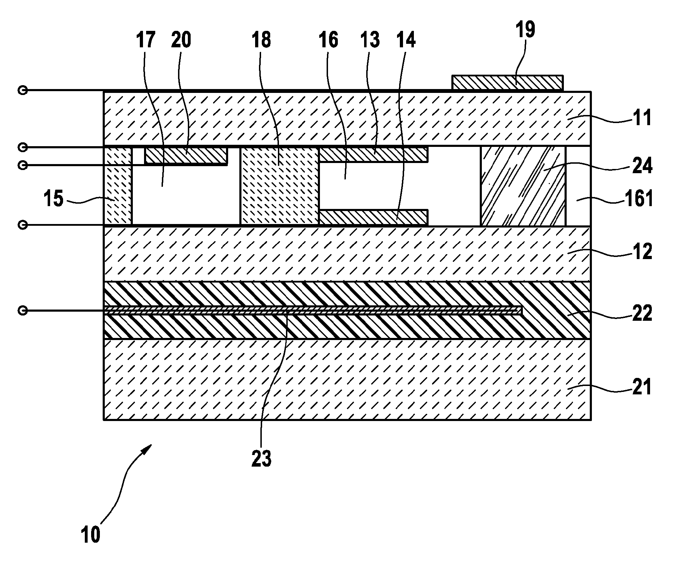

Die Erfindung ist anhand eines in den Zeichnungen dargestellten Ausführungsbeispiels in der nachfolgenden Beschreibung näher erläutert. Dabei zeigt die Zeichnung in schematischer Darstellung einen Längsschnitt eines Sensorelements für einen Gassensor zur Bestimmung des Ammoniakgehalts in einem Messgas.

Das in der Zeichnung im Längsschnitt dargestellte Sensorelement für einen Gassensor zur Bestimmung der Konzentration von Ammoniak als Beispiel für eine oxidierbare Gaskomponente in einem Messgas, vorzugsweise im Abgas einer Brennkraftmaschine, weist einen planaren Keramikkörper 10 auf, der aus einer Mehrzahl von keramischen Folien zusammengesetzt ist, die jeweils eine sauerstoffionenleitende Festelektrolytschicht aus einem Festelektrolytmaterial, wie beispielsweise yttriumstabilisiertes oder -teilstabilisiertes Zirkoniumdioxid (ZrO₂), bilden. Die integrierte Form des planaren Keramikkörpers 10 wird durch Zusammenlaminieren der mit Funktionsschichten bedruckten, keramischen Folien und anschließendem Sintern der laminierten Struktur in an sich bekannter Weise hergestellt. Auf einer ersten, oberen Keramikfolie 11 ist auf voneinander abgekehrten Oberflächen eine Außenelektrode 19 und eine erste Messelektrode 13 aufgebracht, wobei die Außenelektrode 19 unmittelbar dem Messgas ausgesetzt ist. Auf der der Keramikfolie 11 zugekehrten, oberen Folienfläche einer zweiten Keramikfolie 12 ist eine zweite Messelektrode 14 der der ersten Messelektrode 13 gegenüberliegend aufgebracht. Zwischen der ersten und zweiten Keramikfolie 11, 12 ist eine Schicht 15 aus Festelektrolytmaterial angeordnet, in der einerseits ein Messraum 16 und andererseits ein Referenzgaskanal 17 ausgebildet sind. Messraum 16 und Referenzgaskanal 17 sind durch eine Trennwand 18 gasdicht voneinander getrennt. Der Messraum 16 hat einen Messgaszutritt 161 für das den Keramikkörper 10 umgebende Messgas und nimmt die beiden Messelektroden 13, 14 auf. Die Messelektroden 13, 14 sind keramische oder metallische Mischpotenzialelektroden, die katalytisch inaktiv sind. Die metallischen Mischpotenzialelektroden bestehen z.B. aus einer Platin/Gold-Legierung, aber auch Legierungen mit Palladium, Silber oder Ruthenium sind geeignet. Alternativ kann eine der Messelektroden 13, 14 als reine Platinelektrode ausgeführt sein, die damit katalytisch wirksam ist. Der Referenzgaskanal 17 mündet vorzugsweise in der Atmosphäre, so dass eine im Referenzgaskanal 17 angeordnete, auf der Oberfläche der ersten Keramikfolie 11 (alternativ auf der Oberfläche der zweiten Keramikfolie 12) angeordnete Referenzelektrode 20 mit Umgebungsluft beaufschlagt ist. Zwischen der zweiten Keramikfolie 12 und einer dritten Keramikfolie 21 ist ein in einer elektrischen Isolierung 22 eingebetteter, elektrischer Heizer 23 angeordnet, der zum Erwärmen des Sensorelements auf Betriebstemperatur dient. Im Messgaszutritt 161 ist ein regenerierbarer Stickoxid-Speicher 24 aus porösem Material angeordnet, durch den das über den Messgaszutritt 161 in den Messraum 16 eintretende Messgasvolumen hindurchströmt. Im Stickoxid-Speicher 24 werden die im Messgasvolumen enthaltenen Stickoxide absorbiert. Hierzu weist das poröse Material des Stickoxid-Speichers 24 eine bariumhaltige Speicherkomponente und eine Edelmetallkomponente auf. Letztere dient als Katalysator für die Oxidation von Stickstoffmonoxid (NO), da das bei der Oxidation entstehende Stickstoffdioxid besser von der Speicherkomponente absorbiert werden kann. Für die Speicherkomponente finden Metalloxide bzw. Gemische von Metalloxiden, insbesondere Oxide von Alkali, Erdalkali oder Seltenerdmetalle Verwendung, wobei bevorzugt Bariumoxid, aber auch Bariumkarbonat, eingesetzt wird. Als Edelmetall für die Edelmetallkomponente können Platin, Palladium, Rhodium oder Mischungen bzw. Legierung davon verwendet werden. Der so aufgebaute Stickoxid-Speicher 24 weist seine optimale Speicherfähigkeit in einem Temperaturbereich zwischen 200°C und 400°C auf. Um die entsprechende Temperatur am Stickoxid-Speicher 24 einzustellen, ist der elektrische Heizer 23 räumlich so im Keramikkörper 10 angeordnet, dass er einerseits im Messraum 16 eine Betriebstemperatur von ca. 500°C und andererseits im Messgaszutritt 161 die optimale Speichertemperatur des Stickoxid-Speichers 24 einstellt. Die Speicherung des in dem zum Messraum 16 strömenden Messgasvolumens enthaltenen Stickoxiden im Stickoxid-Speicher 24 erfolgt dadurch, dass das Bariumoxid oder das Bariumkarbonat im Stickoxid-Speicher 24 in Bariumnitrat (Ba(NO₃)₂) umgewandelt wird. Der beschriebene Gassensor kann - nichterfindungsgemàss - außer für Ammoniak - auch zur Bestimmung der Konzentration von anderen oxidierbaren Gaskomponenten im Messgas eingesetzt werden. So kann auch die Konzentration von Wasserstoff oder Kohlenwasserstoff gemessen werden.

Zum Messen der Ammoniakkonzentration im Messgas wird an die beiden Messelektroden 13, 14 eine Spannung angelegt, die z.B. zwischen 0 und 1V liegt. Der Strom und/oder die Spannung zwischen den Messelektroden 13, 14 wird als Maß für die Konzentration an Ammoniak im Messgas ausgewertet.

Alternativ kann eine der beiden Messelektroden 13, 14 entfallen und zum Messen der Ammoniakkonzentration die verbliebene Messelektrode 13 bzw. 14 und die Referenzelektrode 20 herangezogen werden. In diesem Fall ist der Strom oder die Spannung zwischen Referenzelektrode 20 und Messelektrode 13 ein Maß für die Konzentration des Ammoniaks im Messgas.

Mit Referenzelektrode 20 und Außenelektrode 19 kann zusätzlich der Sauerstoffgehalt im Messgas wie bekannt gemessen werden.

Verzichtet man auf die Option einer Messung der Sauerstoffkonzentration im Messgas, so können Referenzelektrode 20 und Referenzgaskanal 17 entfallen, doch lässt sich durch Berücksichtigung der Sauerstoffkonzentration das Messergebnis der Ammoniakmessung verbessern.

Ist die Speicherkapazität des Stickoxid-Speichers 24 erschöpft, d.h. das gesamte zur Verfügung stehende Bariumoxid bzw. Bariumkarbonat in Bariumnitrat umgewandelt, so wird der Stickoxid-Speicher 24 regeneriert, und zwar dadurch ,dass der Stickoxid-Speicher 24 kurzzeitig mittels des elektrischen Heizers 23 einer Temperatur größer 500°C ausgesetzt wird. Bei dieser Temperatur zersetzt sich das Bariumnitrat zu Bariumoxid.

Zur Regenerierung des Stickoxid-Speichers 24 kann alternativ im Messraum 16 ein Fettgas erzeugt werden, indem zwischen einer der beiden Messelektroden 13, 14 und der Außenelektrode 19 eine Spannung angelegt wird. In diesem Fall wird Wasser (H₂O) und Kohlendioxid (CO₂), die in dem im Messraum 16 vorhandenen Messgasvolumen enthalten sind, in Kohlenmonoxid (CO) bzw. Wasserstoff (H₂) und Sauerstoff zersetzt, und die durch Elektronenaufnahme gebildeten freien Sauerstoffionen werden aus dem Messraum 16 gepumpt. Im Messraum 16 bildet sich Fettgas (λ<1), das mit dem Bariumnitrat reagiert und dieses in BaO bzw. BaCO₃ umwandelt.

Zur Regeneration des Stickoxid-Speichers 24 kann auch kurzzeitig die Brennkraftmaschine in einen Zustand gefahren werden, in dem im Abgas Bestandteile an H₂ und CO auftreten, ohne dass ein fettes Gasgemisch vorliegt.

## Patentansprüche

1. Verfahren zur Bestimmung der Konzentration von Ammoniak in einem Messgas, insbesondere im Abgas einer Brennkraftmaschine, mittels eines Sensorelements für einen Gassensor mit mindestens einer von Messgasvolumina beaufschlagten Messelektrode (13, 14), **dadurch gekennzeichnet, dass** die die Messelektrode (13, 14) beaufschlagenden Messgasvolumina von dem Sensorelement umfassten Mitteln zur Absorption von Stickoxiden ausgesetzt sind.

2. Verfahren nach Anspruch 1, **dadurch gekennzeichnet, dass** die Messgasvolumia durch die Absorptionsmittel hindurchgeführt sind.

3. Verfahren nach Anspruch 1 oder 2, **dadurch gekennzeichnet, dass** die Absorptionsmittel einen regenerierbaren Stickoxid-Speicher (24) aufweisen.

4. Verfahren nach Anspruch 3, **dadurch gekennzeichnet, dass** der Stickoxid-Speicher (24) poröses Material mit einer bariumhaltigen Speicherkomponente aufweist.

5. Verfahren nach Anspruch 4, **dadurch gekennzeichnet, dass** die Speicherkomponente Metalloxide oder Gemische von Metalloxiden, vorzugsweise Oxide oder Gemische von Oxiden der Alkalimetalle, hier vorzugsweise Natrium oder Kalium, der Erdalkalimetalle, hier vorzugsweise Barium, und/oder der Seltenerdmetalle, hier vorzugsweise Lanthan, aufweist.

6. Verfahren nach Anspruch 3 oder 4, **dadurch gekennzeichnet, dass** der Stickoxid-Speicher (24) eine katalytisch aktive Metallkomponente, vorzugsweise Edelmetallkomponente, zur Oxidation von Stickstoffmonoxid aufweist, die vorzugsweise Platin, Palladium, Rhodium oder Mischungen oder Legierungen davon enthält.

7. Verfahren nach einem der Ansprüche 1 bis 6, **dadurch gekennzeichnet, dass** die mindestens eine Messelektrode (13, 14) aus einem katalytisch inaktiven Material besteht, das vorzugsweise Gold, Palladium, Silber oder Ruthenium enthält oder eine Gold/Platin-Legierung ist.

8. Verfahren nach einem der Ansprüche 3 bis 7, **dadurch gekennzeichnet, dass** zur Regeneration des Stickoxid-Speichers (24) dieser kurzzeitig einer Temperatur größer 500°C aussetzbar ist.

9. Verfahren nach einem der Ansprüche 3 bis 7, **dadurch gekennzeichnet, dass** zur Regeneration des Stickoxid-Speichers (24) Mittel zur Beeinflussung des zwischen Stickoxid-Speicher (24) und Messelektrode (13, 14) vorhandenen Messgasvolumens vorgesehen sind, die in der Weise wirken, dass im Messgasvolumen Anteile von Wasserstoff und Kohlenmonoxid entstehen.

10. Verfahren nach einem der Ansprüche 1 bis 9, **dadurch gekennzeichnet, dass** die mindestens eine Messelektrode (13, 14) in einem einen Messgaszutritt (161) aufweisenden Messraum (16), der zumindest teilweise von einem sauerstoffionenleitenden Festelektrolyten umgeben ist, und der Stickoxid-Speicher (24) im Messgaszutritt (161) angeordnet ist.

11. Verfahren nach Anspruch 10, **dadurch gekennzeichnet, dass** ein die Betriebstemperatur für die mindestens eine Messelektrode (13, 14) einstellender, elektrischer Heizer (23) vorgesehen ist, der relativ zu Messraum (16) und Messgaszutritt (161) so angeordnet ist, dass bei einer an der mindestens einen Messelektrode (13, 14) herrschenden Betriebstemperatur der Stickoxid-Speicher (24) seine optimale Speichertemperatur aufweist, die vorzugsweise in einem Bereich zwischen 200°C und 400°C liegt.

12. Verfahren nach Anspruch 10 oder 11, **dadurch gekennzeichnet, dass** der Messraum (16) von einer sauerstoffionenleitenden Festelektrolytschicht (11) begrenzt ist, auf deren vom Messraum (16) abgekehrten Seite eine dem Messgas aussetzbare Außenelektrode (19) angeordnet ist.

13. Verfahren nach Anspruch 12, **dadurch gekennzeichnet, dass** zur Regeneration des Stickoxid-Speichers (24) an die Außenelektrode (19) und die mindestens einen Messelektrode (13, 14) eine solche Spannung anlegbar ist, dass Sauerstoff aus dem Messraum (16) gepumpt wird.

14. Verfahren nach einem der Ansprüche 10 bis 13, **dadurch gekennzeichnet, dass** eine Referenzelektrode (20) vorgesehen ist, die in einem gegenüber dem Messraum (16) gasdicht abgeschotteten Referenzgaskanal (17) angeordnet ist.

15. Verfahren nach einem der Ansprüche 10 bis 14, **dadurch gekennzeichnet, dass** im Messraum (16) eine zweite Messelektrode (14) angeordnet ist und dass an den beiden Messelektroden (13, 14) eine elektrische Spannung anlegbar und der Strom oder die Spannung zwischen den Messelektroden (13, 14) ein Maß für die Konzentration der Gaskomponente im Messgas ist.

16. Verfahren nach Anspruch 14, **dadurch gekennzeichnet, dass** zwischen Referenzelektrode (20) und der mindestens einen Messelektrode (13, 14) eine elektrische Spannung anlegbar ist und die Spannung oder der Strom zwischen Referenzelektrode (20) und Messelektrode (13, 14) ein Maß für die Konzentration der Gaskomponente ist.

17. Verfahren nach einem der Ansprüche 14 bis 16, **dadurch gekennzeichnet, dass** zur Messung des Sauerstoffgehalts im Messgas an Referenzelektrode (20) und Außenelektrode (19) eine Spannung anlegbar und das zwischen Referenzelektrode (20) und Außenelektrode (19) auftretende Spannungspotenzial ein Maß für die Sauerstoffkonzentration im Messgas ist.

## Claims

1. Method for determining the concentration of ammonia in a measurement gas, in particular in the exhaust gas from an internal combustion engine, by means of a sensor element for a gas sensor having at least one measurement electrode (13, 14) which is acted on by volumes of measurement gas, **characterized in that** the volumes of measurement gas which act on the measurement electrode (13, 14) are exposed to means for absorbing nitrogen oxides, which means are included in the sensor element.

2. Method according to Claim 1, **characterized in that** the volumes of measurement gas are routed through the absorption means.

3. Method according to Claim 1 or 2, **characterized in that** the absorption means have a nitrogen oxide store (24) which can be regenerated.

4. Method according to Claim 3, **characterized in that** the nitrogen oxide store (24) comprises porous material with a barium-containing storage component.

5. Method according to Claim 4, **characterized in that** the storage component comprises metal oxides or mixtures of metal oxides, preferably oxides or mixtures of oxides of the alkali metals, in this case preferably sodium or potassium, of the alkaline-earth metals, in this case preferably barium, and/or of the rare-earth metals, in this case preferably lanthanum.

6. Method according to Claim 3 or 4, **characterized in that** the nitrogen oxide store (24) has a catalytically active metal component, preferably stainless-metal component, for oxidizing nitrogen monoxide, the said catalytically active metal component preferably containing platinum, palladium, rhodium or mixtures or alloys thereof.

7. Method according to one of Claims 1 to 6, **characterized in that** the at least one measurement electrode (13, 14) is composed of a catalytically inactive material which preferably contains gold, palladium, silver or ruthenium or is a gold/platinum alloy.

8. Method according to one of Claims 3 to 7, **characterized in that**, in order to regenerate the nitrogen oxide store (24), the said nitrogen oxide store can be briefly exposed to a temperature of greater than 500°C.

9. Method according to one of Claims 3 to 7, **characterized in that**, in order to regenerate the nitrogen oxide store (24), means for influencing the measurement gas volume which is present between the nitrogen oxide store (24) and the measurement electrode (13, 14) are provided, the said means acting in such a way that proportions of hydrogen and carbon monoxide are produced in the measurement gas volume.

10. Method according to one of Claims 1 to 9, **characterized in that** the at least one measurement electrode (13, 14) in a measurement space (16), which has a measurement gas inlet (161) and is at least partially surrounded by an oxygen ion-conducting solid electrolyte, and the nitrogen oxide store (24) is arranged in the measurement gas inlet (161).

11. Method according to Claim 10, **characterized in that** an electric heater (23) which sets the operating temperature for the at least one measurement electrode (13, 14) is provided, the said electric heater being arranged relative to the measurement space (16) and the measurement gas inlet (161) such that, at a operating temperature which prevails at the at least one measurement electrode (13, 14), the nitrogen oxide store (24) is at its optimum storage temperature which is preferably in a range of between 200°C and 400°C.

12. Method according to Claim 10 or 11, **characterized in that** the measurement space (16) is delimited by an oxygen ion-conducting solid electrolyte layer (11), an outer electrode (19) which can be exposed to the measurement gas being arranged on that side of the said solid electrolyte layer which is averted from the measurement space (16).

13. Method according to Claim 12, **characterized in that**, in order to regenerate the nitrogen oxide store (24), a voltage such that oxygen is pumped out of the measurement space (16) can be applied to the outer electrode (19) and the at least one measurement electrode (13, 14).

14. Method according to one of Claims 10 to 13, **characterized in that** a reference electrode (20) is provided, the said reference electrode being arranged in a reference gas duct (17) which is sealed off from the measurement space (16) in a gas-tight manner.

15. Method according to one of Claims 10 to 14, **characterized in that** a second measurement electrode (14) is arranged in the measurement space (16), and **in that** an electrical voltage can be applied to the two measurement electrodes (13, 14), and the current or the voltage between the measurement electrodes (13, 14) is a measure of the concentration of the gas component in the measurement gas.

16. Method according to Claim 14, **characterized in that** an electrical voltage can be applied between the reference electrode (20) and the at least one measurement electrode (13, 14), and the voltage or the current between the reference electrode (20) and the measurement electrode (13, 14) is a measure of the concentration of the gas component.

17. Method according to one of Claims 14 to 16, **characterized in that**, in order to measure the oxygen content in the measurement gas, a voltage can be applied to the reference electrode (20) and the outer electrode (19), and the voltage potential which is produced between the reference electrode (20) and the outer electrode (19) is a measure of the oxygen concentration in the measurement gas.

## Revendications

1. Procédé de détermination de la concentration d'ammoniac dans un gaz de mesure, notamment dans les gaz d'échappement d'un moteur à combustion interne, au moyen d'un élément de détection pour un capteur de gaz comprenant au moins une électrode de mesure (13, 14) chargée avec des volumes de gaz de mesure,
**caractérisé en ce que** les volumes de gaz de mesure qui chargent l'électrode de mesure (13, 14) sont exposés à des moyens entourant l'élément de détection et destinés à l'absorption d'oxydes d'azote.

2. Procédé selon la revendication 1, **caractérisé en ce que** les volumes de gaz de mesure sont acheminés à travers les moyens d'absorption.

3. Procédé selon la revendication 1 ou 2, **caractérisé en ce que** les moyens d'absorption possèdent un accumulateur d'oxyde d'azote (24) régénérable.

4. Procédé selon la revendication 3, **caractérisé en ce que** l'accumulateur d'oxyde d'azote (24) possède un matériau poreux comprenant un composant d'accumulation contenant du baryum.

5. Procédé selon la revendication 4, **caractérisé en ce que** le composant d'accumulation possède des oxydes métalliques ou des mélanges d'oxydes métalliques, de préférence des oxydes ou des mélanges d'oxydes des métaux alcalins, ici de préférence du sodium ou du potassium, des métaux alcalinoterreux, ici de préférence du baryum, et/ou des terres rares, ici de préférence du lanthane.

6. Procédé selon la revendication 3 ou 4, **caractérisé en ce que** l'accumulateur d'oxyde d'azote (24) possède un composant métallique actif du point de vue catalytique, de préférence un composant à base de métal précieux, pour l'oxydation du monoxyde d'azote, lequel contient de préférence du platine, du palladium, du rhodium ou encore des mélanges ou des alliages de ceux-ci.

7. Procédé selon l'une des revendications 1 à 6, **caractérisé en ce que** l'au moins une électrode de mesure (13, 14) se compose d'un matériau inactif du point de vue catalytique, lequel contient de préférence de l'or, du palladium, de l'argent ou du ruthénium ou est un alliage d'or et de platine.

8. Procédé selon l'une des revendications 3 à 7, **caractérisé en ce que**, pour la régénération de l'accumulateur d'oxyde d'azote (24), celui-ci peut être exposé pendant une courte durée à une température supérieure à 500 °C.

9. Procédé selon l'une des revendications 3 à 7, **caractérisé en ce que**, pour la régénération de l'accumulateur d'oxyde d'azote (24), il existe des moyens destinés à influencer le volume de gaz de mesure présent entre l'accumulateur d'oxyde d'azote (24) et l'électrode de mesure (13, 14), lesquels agissent de telle sorte que des portions d'hydrogène et de monoxyde de carbone se produisent dans le volume de gaz de mesure.

10. Procédé selon l'une des revendications 1 à 9, **caractérisé en ce que** l'au moins une électrode de mesure (13, 14) est disposée dans un espace de mesure (16), lequel possède une entrée de gaz de mesure (161) et est au moins partiellement entouré par un électrolyte solide conduisant les ions d'oxygène, et l'accumulateur d'oxyde d'azote (24) est disposé dans l'entrée de gaz de mesure (161).

11. Procédé selon la revendication 10, **caractérisé en ce qu'**il existe un élément chauffant électrique (23) qui règle la température de service pour l'au moins une électrode de mesure (13, 14), lequel est disposé de telle sorte par rapport à l'espace de mesure (16) et à l'entrée de gaz de mesure (161) que l'accumulateur d'oxyde d'azote (24) présente sa température d'accumulation optimale, laquelle se trouve de préférence dans une plage comprise entre 200 °C et 400 °C, lorsqu'une température de service règne au niveau de l'au moins une électrode de mesure (13, 14).

12. Procédé selon la revendication 10 ou 11, **caractérisé en ce que** l'espace de mesure (16) est délimité par une couche d'électrolyte solide (11) conduisant les ions d'oxygène, sur le côté à l'opposé de l'espace de mesure (16) de laquelle est disposée une électrode extérieure (19) pouvant être exposée au gaz de mesure.

13. Procédé selon la revendication 12, **caractérisé en ce que**, pour la régénération de l'accumulateur d'oxyde d'azote (24), une tension qui permet le pompage de l'oxygène hors de l'espace de mesure (16) peut être appliquée à l'électrode extérieure (19) et à l'au moins une électrode de mesure (13, 14).

14. Procédé selon l'une des revendications 10 à 13, **caractérisé en ce qu'**il existe une électrode de référence (20) qui est disposée dans un canal à gaz de référence (17) à cloisonnement hermétique au gaz par rapport à l'espace de mesure (16).

15. Procédé selon l'une des revendications 10 à 14, **caractérisé en ce qu'**une deuxième électrode de mesure (14) est disposée dans l'espace de mesure (16) et **en ce qu'**une tension électrique peut être appliquée aux deux électrodes de mesure (13, 14) et le courant ou la tension entre les électrodes de mesure (13, 14) est une mesure de la concentration de la composante gazeuse dans le gaz de mesure.

16. Procédé selon la revendication 14, **caractérisé en ce qu'**une tension électrique peut être appliquée entre l'électrode de référence (20) et l'au moins une électrode de mesure (13, 14) et la tension ou le courant entre l'électrode de référence (20) et l'électrode de mesure (13, 14) est une mesure de la concentration de la composante gazeuse.

17. Procédé selon l'une des revendications 14 à 16, **caractérisé en ce qu'**une tension peut être appliquée à l'électrode de référence (20) et à l'électrode extérieure (19) pour la mesure de la teneur en oxygène et le potentiel de tension produit entre l'électrode de référence (20) et l'électrode extérieure (19) est une mesure de la concentration d'oxygène dans le gaz de mesure.
